# EUROPEAN PATENT SPECIFICATION

(11) **EP 3 142 723 B1**
(45) Date of publication and mention of the grant of the patent: **04.07.2018**
(21) Application number: 15728626.1
(22) Date of filing: 11.05.2015
(51) Int. Cl.: A61M 1/30, A61M 1/34

(54) **SINGLE NEEDLE HEMO(DIA)FILTRATION APPARATUS**
EINNADELIGE HÄMO(DIA)FILTRIERUNGSVORRICHTUNG
APPAREIL D'HÉMO(DIA)FILTRATION À AIGUILLE UNIQUE

(30) Priority: 13.05.2014 IT MO20140129
(43) Date of publication of application: 22.03.2017
(73) Proprietor: Zanotti, Monica, 41124 Modena (IT)
(72) Inventor: Zanotti, Monica, 41124 Modena (IT)
(74) Representative: Villanova, Massimo
(86) International application number: PCT/IB2015/053448
(87) International publication number: WO 2015/173713

(56) References cited:
- EP-A1- 0 122 604
- GB-A- 2 141 936
- US-A- 5 047 147
- US-A1- 2011 178 452
- US-A1- 2012 029 409

## Description

### Background of the invention

The invention relates to a single needle extracorporeal blood treatment apparatus for hemofiltration or hemodiafiltration treatments.

Specifically, but not exclusively, the invention can be used for hemo(dia)filtration treatments with relatively low extracorporeal blood flow rates (e.g. for treatments on children or on a patient with restricted blood access).

In particular, it refers to an apparatus comprising a blood treatment membrane device, a single needle extracorporeal blood circuit and means for controlling the apparatus cyclically with a phase of withdrawal from the patient and a phase of return to the patient, wherein during the return phase a fraction of the flow that comes from the membrane device goes to the patient through the single needle while the remaining fraction returns to the membrane device.

As described in the publications US 7780848 (hemodiafiltration), US 2011/0178452 (hemodiafiltration) and US 8394047 (pure ultrafiltration) during the return (venous) phase, the fluid that flows into the venous line has a fraction that goes back to the patient, while the remaining fraction recirculates returning to the membrane device through the arterial line. The publication EP 832656 shows another example of single needle hemo(dia)filtration apparatus.

US 2011/0178452 A1 discloses an apparatus as in the preamble of claim 1.

### Summary of the invention

An aim of the invention is to provide a single-needle blood treatment apparatus for hemo(dia)filtration.

An advantage is to make a single-needle blood treatment apparatus that is simple and economical.

An advantage is to realize an extracorporeal blood treatment apparatus with extracorporeal blood flow rate that is relatively low.

An advantage is to allow a continuous flow in the blood chamber of the blood treatment membrane device.

An advantage is to avoid an excessive hemoconcentration in the blood treatment membrane device.

An advantage is to allow a single needle treatment without interrupting the flow in the extracorporeal blood circuit, especially without the use of clamps.

An advantage is to allow a single needle hemo(dia)filtration with a continuous ultrafiltration flow.

These aims and advantages, and others, are achieved by the apparatus according to one or more of the claims below.

In one embodiment, a single needle hemo(dia)filtration apparatus comprises a blood treatment membrane device, pumping means controlled to continuously circulate fluid in a closed annular portion of extracorporeal blood circuit, ultrafiltration means controlled to generate a continuous flow of ultrafiltration in the membrane device, and means for infusing a substitution fluid in the extracorporeal circuit upstream and/or downstream of the membrane device, said means being controlled in such a way as to generate, cyclically, an arterial phase of withdrawing from the patient, wherein the flow of substitution fluid is less than the ultrafiltration flow, and a venous phase of returning to the patient, wherein the flow of substitution fluid is greater than the ultrafiltration flow.

Further characteristics and advantages of the present invention will better emerge from the detailed description that follows.

### Brief description of the drawings

The invention can be better understood and implemented with reference to the attached drawings which illustrate some non-exhaustive examples of implementation.
Figure 1 is a schematic drawing of a first example of single needle hemo(dia)filtration apparatus in an arterial phase.
Figure 2 shows the apparatus of figure 1 in a venous phase.
Figure 3 is a schematic drawing of a second example of single needle hemo(dia)filtration apparatus in an arterial phase.
Figure 4 shows the apparatus of figure 3 in a venous phase.

### Detailed description

Referring to the above figures, analogous items have been indicated, where possible, with the same reference number.

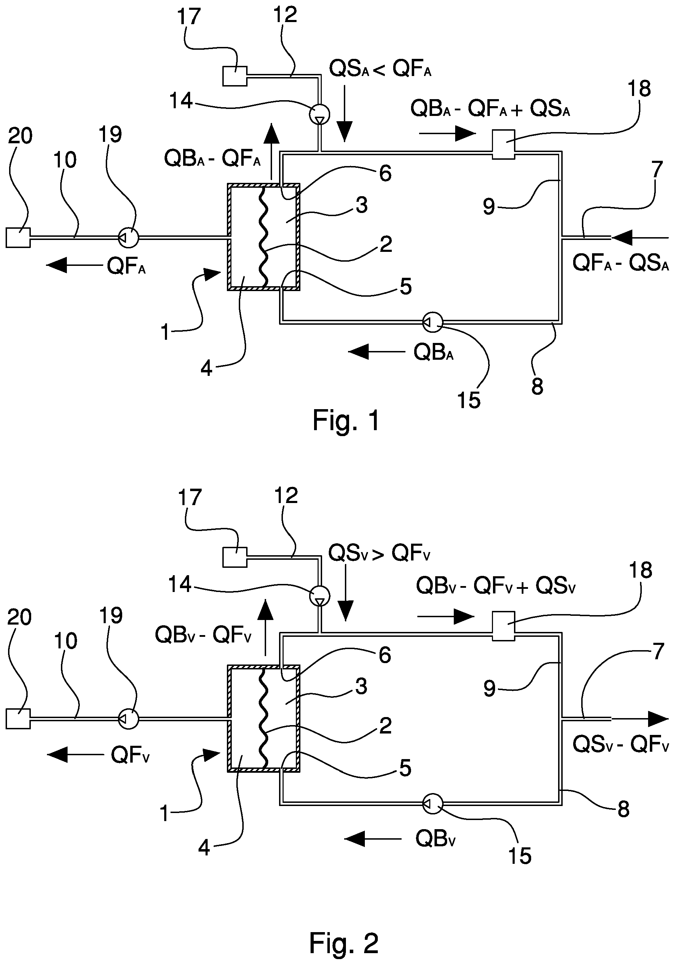

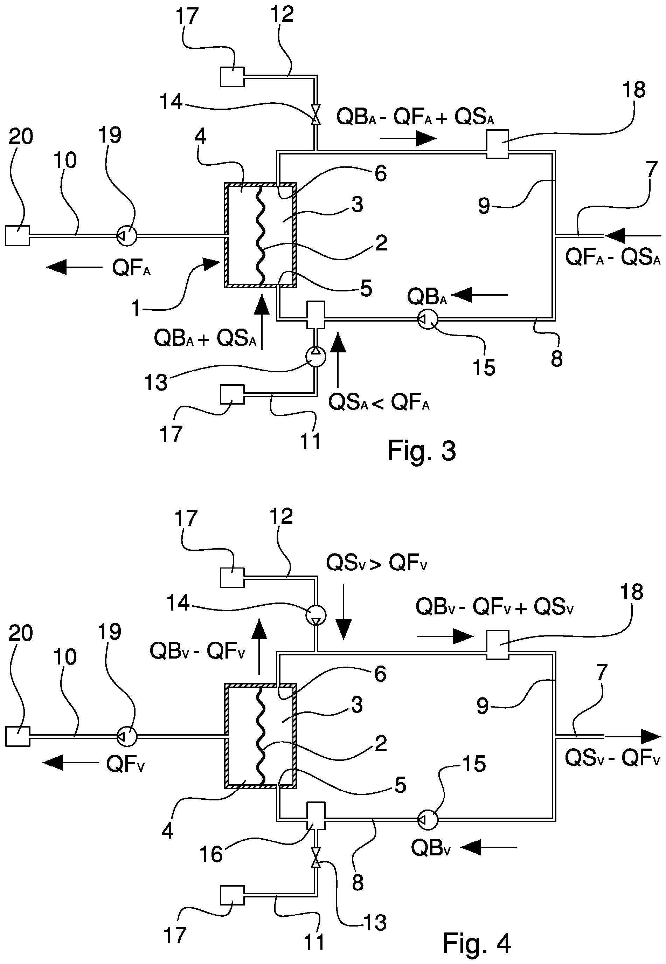

In figures 1 to 4, 1 denotes a blood treatment membrane device (such as a hemofilter or hemodiafilter), 2 a semipermeable membrane of the device 1, 3 a first chamber of the device 1 (blood chamber forming part of an extracorporeal blood circuit), 4 a second chamber of the device 1 (fluid chamber forming part of a fluid circuit in which a fresh treatment fluid could flow, in particular a dialysis fluid). The first chamber 3 (blood chamber) has a first opening 5 (inlet and/or outlet) and a second opening 6 (outlet and/or inlet) for the passage of extracorporeal blood.

Each hemodiafiltration apparatus of figures 1 to 4 has a single needle extracorporeal blood circuit having a three-way junction area, a patient line 7 extended between the junction area and a single needle patient access (not shown), a first line 8 extended between the junction area and the first opening 5 and a second line 9 extended between the junction area and the second opening 6.

Each apparatus has a fluid circuit having at least one exit line 10 or drain line to drain spent fluid from the second chamber 4 to a drain 20.

Each apparatus has an infusion circuit to supply a substitution fluid to the blood circuit. The infusion circuit may have at least one infusion line (pre-dilution or post-dilution) which flows into the first line 8 or second line 9.

Each apparatus may have first actuator means for controlling at least a first flow rate QB of fluid (blood flow) in the extracorporeal blood circuit, second actuator means for controlling at least a second flow rate QS (infusion flow) in the infusion circuit to supply the extracorporeal circuit with the substitution fluid, and third actuator means (e.g. comprising at least one ultrafiltration pump 19) for controlling at least a third flow rate QF of ultrafiltration from the first chamber 3 to the second chamber 4 through the semipermeable membrane 2.

Each apparatus may comprise control means for controlling the first, second and third actuator means, wherein such control means may comprise means for accomplishing one or more arterial phases of withdrawal from the patient, wherein the third flow rate QF_{A} of ultrafiltration is greater than the second flow rate QS_{A} of substitution fluid, alternated with one or more venous phases of return to the patient, wherein the third flow rate QF_{V} of ultrafiltration is less than the second flow rate QS_{V} of substitution fluid.

The above control means, which is configured to carry out one or more arterial phases alternated with one or more venous phases, may comprise, in particular, a programmable electronic control unit provided with computer program instructions.

The third flow rate Q_{F} of ultrafiltration may be not null (directed from the first chamber 3 to the second chamber 4) in one or more arterial phases and/or in one or more venous phases, in such a way as to continuously ultrafilter from the first chamber 3 to the second chamber 4 during the treatment (entirely or at least partially).

The first flow rate Q_{B} of fluid may be non-zero in one or more arterial phases and/or in one or more venous phases, so as to generate a one-way continuous flow in a closed annular path that includes the first line 8, the second line 9 and the first chamber 3.

The first flow rate Q_{B} of fluid may be, in particular, more than double (or more than triple) of the third flow rate Q_{F} of ultrafiltration in one or more of (or all) the arterial phases and/or in one or more of (or all) the venous phases.

The infusion circuit may comprise a first dilution line 11 that flows into the first line 8 and/or a second dilution line 12 that flows into the second line 9.

The second actuator means for infusion may comprise, for example, means for generating a first dilution flow rate Qpre (e.g. an infusion pump 13) in the first dilution line 11 and means for generating a second dilution flow rate Qpost (e.g. an infusion pump 14) in the second dilution line 12. It is possible to control the second actuator means so that the second dilution flow rate Qpost (or post-dilution flow rate) is greater in the venous phases than in the arterial phases and/or so that the first dilution flow rate Qpre (or pre-dilution flow rate) is less in the venous phases than in the arterial phases.

The first actuator means may comprise, for example, an only-one blood pump 15 placed in the first line 8 (or in the second line 9).

In particular the first actuator means may comprise an only-one blood pump 15 placed in the first line 8 to pump from the junction area to the first chamber 3, and the second actuator means may be arranged to infuse substitution fluid into a second dilution line 12 (post-dilution) which flows into, for example, the second line 9.

The first actuator means may comprise actuator means of the reversible type to circulate fluid in both directions.

The blood pump 15 and/or the infusion pump 13 and/or the infusion pump 14 and/or the ultrafiltration pump 19 may comprise, in particular, a pump of occlusive type, e.g. a peristaltic pump.

The fluid circuit may comprise, in particular, a supply line (not shown) to supply fresh fluid to the second chamber 4 (hemodiafiltration). The supply line to supply fresh fluid may be absent or locked (hemofiltration).

The infusion circuit may be connected to a source 17 of substitution fluid, in which such a source 17 may include a on-line fluid preparation system from water and concentrates (with means for depurating fluid, e.g. one or more ultrafilters) and/or a system with container/s of the batch type.

The infusion circuit may comprise at least one first dilution line (pre-dilution line) and at least one second dilution line (post-dilution line) connected to a common dilution line in branch relationship: in this case a pump may be arranged to generate a fluid flow rate in the common dilution line and/or at least two distinct pumps, one pump arranged in the post-dilution line and the other pump arranged in the pre-dilution line.

The extracorporeal blood circuit may comprise, in particular, elements normally present in an extracorporeal blood circuit for hemodialysis or hemo(dia)filtration of known type, such as (on the first line 8 and/or on the second line 9): pierceable access means and/or means for connecting to a pressure sensor and/or means for detecting the presence of air and/or means for injecting an anticoagulant, etc.

The hemo(dia)filtration apparatus may comprise sensors means for detecting the concentration of fluid in the extracorporeal blood circuit. Such concentration sensor means may comprise, for example, a hematocrit sensor. Such concentration sensor means may be placed, in particular, on the first line 8 of the blood circuit. The hemo(dia)filtration apparatus may comprise sensor means for detecting the pressure of fluid in the extracorporeal blood circuit. Such pressure sensor means may be placed on the first line 8 and/or the second line 9 and/or the exit line 10 of spent fluid and/or the supply line of fresh fluid and/or the first dilution line 11 and/or the second dilution line 12.

The hemo(dia)filtration apparatus may comprise gas-liquid separation means arranged in the blood circuit. Such separation means may comprise at least one expansion chamber 18 arranged in the blood circuit, particularly in the second line 9, to separate air from the fluid in the circuit.

The hemo(dia)filtration apparatus may comprise, in particular, a mixing chamber 16 arranged in the first line 8 to mix the pre-dilution flow rate into the fluid.

The hemo(dia)filtration apparatus may comprise fluid balance means, such as means of known type, which may comprise, in particular, scale means and/or flowmeter means and/or variable volume chamber means, etc. The control unit may be programmed to control the apparatus based on a desired patient weight loss.

The hemo(dia)filtration apparatus may comprise a user interface, in particular a graphical interface, for example comprising a touch screen, or any other known type user interface, to allow communication between the user and the programmable electronic control unit of the apparatus.

In use, the electronic control unit can be programmed to perform alternating cycles of arterial phases (taking blood from the patient) and venous phase (returning blood to the patient). In at least one arterial phase, the third flow rate QF_{A} of ultrafiltration may be less than the first flow rate QB_{A} of fluid and greater than the second flow rate QS_{A} of substitution fluid. This will generate a flow rate of withdrawal from the patient equal to QF_{A}-QS_{A} and a one-way continuous flow of fluid (blood and fluid substitution) along the closed-loop path. In at least one venous phase, the third flow rate QF_{V} of ultrafiltration may be greater than zero and less than both the first flow rate QB_{V} of fluid and the second flow rate QS_{V} of substitution fluid. This will generate a flow rate of return to the patient equal to QS_{V}-QF_{V} and a one-way continuous flow of fluid (blood and substitution fluid) in the closed-loop path.

The duration of a (venous and/or arterial) phase may be controlled according to various selectable control modes. For example, it is possible to stop the (venous and/or arterial) phase after a desired period of time. For example, it is possible to set a variable duration of (venous and/or arterial) phases in the course of the treatment. In particular it is possible to provide that the duration of the various (venous and/or arterial) phases is increasing in the course of the treatment: it is possible to program one or more arterial phases having a first set duration in a first period of treatment and one or more arterial phases having a second set duration (greater than the first set duration) in a second period of the same treatment session after the first period. It is possible to program one or more venous phases having a first set duration in a first period of treatment and one or more venous phases having a second set duration (greater than the first set duration) in a second period of the same treatment session after the first period.

It is possible to stop a (venous and/or arterial) phase if the concentration of the liquid measured by sensor means exceeds a set threshold.

It is possible to stop a (venous and/or arterial) phase when the blood volume taken from the patient and/or the blood volume returned to the patient exceeds a set threshold.

It is possible to stop a (venous and/or arterial) phase when the ultrafiltration volume exceeds a set threshold.

It is possible to adopt a control mode that combines two or more of the above-mentioned control modes.

## Claims

1. Hemo(dia)filtration apparatus comprising:
- a blood treatment device (1) having a first chamber (3), a second chamber (4) and a semipermeable membrane (2) that separates said first chamber (3) from said second chamber (4), said first chamber having at least one first opening (5) and one second opening (6);
- a single needle extracorporeal blood circuit comprising a first line (8) connected to said first opening (5) and a second line (9) connected to said second opening (6);
- a fluid circuit having at least one exit line (10) to withdraw spent fluid from said second chamber (4);
- at least one infusion line (11; 12) which flows into said first line (8) and/or said second line (9) to infuse a substitution fluid;
- a first pump (15) arranged to generate a first flow rate (QB) of fluid in said blood circuit;
- a second pump (13; 14) arranged to generate a second flow rate (QS) of substitution fluid in said infusion line (11; 12);
- ultrafiltration means (19) arranged to generate a third flow rate (QF) of ultrafiltration from said first chamber (3) to said second chamber (4) through said semipermeable membrane (2);
- control means arranged to control said first pump (15), said second pump (13; 14) and said ultrafiltration means (19), said control means comprising means for performing withdrawal arterial phases alternated with return venous phases, **characterized in that**:
* in at least one arterial phase, the third flow rate QF_{A} of ultrafiltration is less than the first flow rate QB_{A} of fluid and greater than the second flow rate QS_{A} of substitution fluid, having thereby a flow rate QF_{A}-QS_{A} of withdrawal from patient;
* in at least one venous phase, the third flow rate QF_{V} of ultrafiltration is greater than zero and less than both the first flow rate QB_{V} of fluid and the second flow rate QS_{V} of substitution fluid, having thereby a flow rate QS_{V}-QF_{V} of return to the patient.

2. Apparatus according to claim 1, wherein the second flow rate QS_{A} of substitution fluid in said arterial phase is less than the second flow rate QS_{V} of substitution fluid in said venous phase.

3. Apparatus according to claim 2, wherein the second flow rate QS_{A} of substitution fluid in said arterial phase is non-zero.

4. Apparatus according to any preceding claim, wherein the third flow rate QF_{A} of ultrafiltration in said arterial phase is equal, or nearly equal, to the third flow rate QF_{V} of ultrafiltration in said venous phase, being in the range:
QF_{A} = QF_{V} ± 10%, or QF_{A} = QF_{V} ± 20%, or QF_{A} = QF_{V} ± 30%; and/or
wherein the first flow rate QB_{A} of fluid in said arterial phase is equal, or nearly equal, to the first flow rate QB_{V} of fluid in said venous phase, being in the range:
QB_{A} = QB_{V} ± 10%, or QB_{A} = QB_{V} ± 20%, or QB_{A} = QB_{V} ± 30%.

5. Apparatus according to any preceding claim, wherein said first pump (15) is the only blood pump operating in said blood circuit, arranged or in said first line (8) or in said second line (9).

6. Apparatus according to any preceding claim, wherein: said first pump (15) is the only blood pump operating in said blood circuit in said first line (8) to pump fluid to said first chamber (3); said infusion line comprises a post-dilution line (12) which flows into said second line (9); said second pump (14) is arranged to supply a flow of substitution fluid to said post-dilution line (12).

7. Apparatus according to any preceding claim, wherein said infusion line comprises a post-dilution line (12) which flows into said second line (9) and a pre-dilution line (11) that flows into said first line (8); said control means comprising means for achieving a post-dilution flow rate (Qpost) in said post-dilution line (12) that is greater in said venous phase than in said arterial phase and/or means for achieving a pre-dilution flow rate (Qpre) in said pre-dilution line (11) that is less in said venous phase than in said arterial phase.

8. Apparatus according to any preceding claim, wherein said first pump (15) is reversible for fluid flow in both directions.

9. Apparatus according to any preceding claim, wherein, in said arterial phase, the first flow rate QB_{A} of fluid is greater than twice the third flow rate QF_{A} of ultrafiltration, and/or wherein, in said venous phase, the first flow rate QB_{V} of fluid is greater than twice the third flow rate QF_{V} of ultrafiltration.

10. Apparatus according to any preceding claim, wherein said control means comprises a control mode that is selectable by a user interface of said apparatus, said control mode being based on set durations of arterial phase and/or venous phase, said set durations being variable durations that increase during the same hemo(dia)filtration treatment session so as to have at least one first arterial phase with a first set duration and at least one second arterial phase with a second set duration that is greater than said first set duration, said second arterial phase being after said first arterial phase.

## Patentansprüche

1. Hämo(dia)filtrationsvorrichtung, umfassend:
eine Blutbehandlungsvorrichtung (1) mit einer ersten Kammer (3), einer zweiten Kammer (4) und einer semipermeablen Membran (2), die die erste Kammer (3) von der zweiten Kammer (4) trennt, wobei die erste Kammer mindestens eine erste Öffnung (5) und eine zweite Öffnung (6) aufweist;
einen extrakorporalen Einnadel-Blutkreislauf mit einer ersten Leitung (8), die mit der ersten Öffnung (5) verbunden ist, und einer zweiten Leitung (9), die mit der zweiten Öffnung (6) verbunden ist;
einen Fluidkreislauf mit mindestens einer Auslassleitung (10) zum Abziehen von gebrauchter Flüssigkeit aus der zweiten Kammer (4);
mindestens eine Infusionsleitung (11; 12), die in die erste Leitung (8) und/oder die zweite Leitung (9) fließt, um eine Substitutionsflüssigkeit zu infundieren;
eine erste Pumpe (15), die angeordnet ist, um eine erste Flussrate (QB) von Flüssigkeit in dem Blutkreislauf zu erzeugen;
eine zweite Pumpe (13; 14), die angeordnet ist, um eine zweite Flussrate (QS) von Substitutionsflüssigkeit in der Infusionsleitung (11; 12) zu erzeugen;
Ultrafiltrationsmittel (19), die angeordnet sind, um eine dritte Ultrafiltrationsrate (QF) aus der ersten Kammer (3) in der zweiten Kammer (4) durch die semipermeable Membran (2) zu erzeugen;
Steuermittel, die angeordnet sind, um die erste Pumpe (15), die zweite Pumpe (13; 14) und die Ultrafiltrationsmittel (19) zu steuern, wobei die Steuermittel Mittel zur Durchführung arterieller Rückzugsphasen abwechselnd mit venösen Rückkehrphasen umfassen,
**dadurch gekennzeichnet, daß**:
in mindestens einer arteriellen Phase ist die dritte Flussrate QF_{A} der Ultrafiltration geringer als die erste Flussrate QB_{A} der Flüssigkeit und größer als die zweite Flussrate QS_{A} der Substitutionsflüssigkeit, wodurch sie eine Flussrate QF_{A}-QS_{A} der Entnahme aus dem Patienten aufweist;
in mindestens einer venösen Phase ist die dritte Flussrate QF_{V} der Ultrafiltration größer als Null und kleiner als die erste Flussrate QB_{V} der Flüssigkeit und als die zweite Flussrate QS_{V} der Substitutionsflüssigkeit, wodurch eine Flussrate QS_{V}-QF_{V} zum Patienten zurückkehrt.

2. Vorrichtung nach Anspruch 1, wobei die zweite Flussrate QS_{A} der Substitutionsflüssigkeit in der arteriellen Phase geringer ist als die zweite Flussrate QS_{V} der Substitutionsflüssigkeit in der venösen Phase.

3. Vorrichtung nach Anspruch 2, wobei die zweite Flussrate QS_{A} der Substitutionsflüssigkeit in der arteriellen Phase nicht Null ist.

4. Vorrichtung nach einem der vorhergehenden Ansprüche, wobei die dritte Flussrate QF_{A} der Ultrafiltration in der arteriellen Phase gleich oder nahezu gleich der dritten Flussrate QF_{V} der Ultrafiltration in der venösen Phase ist, die in folgendem Bereich liegt: QF_{A} = QF_{V} ± 10% oder QF_{A} = QF_{V} ± 20% oder QF_{A} = QF_{V} ± 30%; und/oder wobei die erste Flussrate QB_{A} der Flüssigkeit in der arteriellen Phase gleich oder nahezu gleich der ersten Flussrate QB_{V} der Flüssigkeit in der venösen Phase ist, die in folgendem Bereich liegt: QB_{A} = QB_{V} ± 10% oder QB_{A} = QB_{V} ± 20% oder QB_{A} = QB_{V} ± 30%.

5. Vorrichtung nach einem der vorhergehenden Ansprüche, wobei die erste Pumpe (15) die in der ersten Leitung (8) oder in der zweiten Leitung (9) angeordnet ist, die einzige Blutpumpe ist, die in dem Blutkreislauf arbeitet.

6. Vorrichtung nach einem der vorhergehenden Ansprüche, wobei: die erste Pumpe (15) die einzige Blutpumpe ist, die in dem Blutkreislauf in der ersten Leitung (8) arbeitet, um Flüssigkeit zu der ersten Kammer (3) zu pumpen; die Infusionsleitung umfasst eine Post-Verdünnungsleitung (12), die in die zweite Leitung (9) fließt; die zweite Pumpe (14) ist so angeordnet, dass sie der Post-Verdünnungsleitung (12) einen Fluss von Substitutionsflüssigkeit zuführt.

7. Vorrichtung nach einem der vorhergehenden Ansprüche, wobei die Infusionsleitung eine Post-Verdünnungsleitung (12) umfasst, die in die zweite Leitung (9) strömt und eine Vor-Verdünnungsleitung (11) umfasst, die in die erste Leitung (8) strömt; wobei die Steuermittel Mittel zum Erreichen einer Post-Verdünnungsflussrate (Qpost) in der Post-Verdünnungsleitung (12) umfassen, die in der venösen Phase größer ist als in der arteriellen Phase und/oder Mittel zum Erreichen einer Vor-Verdünnungsflussrate (Qpre) in der Vor-Verdünnungsleitung (11), die in der venösen Phase geringer ist als in der arteriellen Phase.

8. Vorrichtung nach einem der vorhergehenden Ansprüche, wobei die erste Pumpe (15) für eine Fluid-Strömung in beiden Richtungen reversibel ist.

9. Vorrichtung nach einem der vorhergehenden Ansprüche, wobei in der arteriellen Phase die erste Flussrate QB_{A} der Flüssigkeit größer als zweimal Doppelte die dritte Flussrate QF_{A} der Ultrafiltration ist, und/oder wobei in der venösen Phase die erste Flussrate QB_{V} der Flüssigkeit größer als zweimal die dritte Flussrate QF_{V} der Ultrafiltration ist.

10. Vorrichtung nach einem der vorhergehenden Ansprüche, wobei die Steuermittel einen Steuermodus aufweisen, der durch eine Benutzerschnittstelle der Vorrichtung auswählbar ist, wobei der Steuermodus auf bestimmten Zeitdauern der arteriellen Phasen und/oder der venösen Phasen basiert, die bestimmten Zeitdauern sind variable Zeitdauern, die während derselben Hämo(dia)filtrationsbehandlungssitzung ansteigen, um mindestens eine erste arterielle Phase mit einer ersten bestimmten Zeitdauer und mindestens eine zweite arterielle Phase mit einer zweiten bestimmten Zeitdauer, die größer als die erste bestimmte Zeitdauer ist, zu haben, wobei die zweite arterielle Phase der ersten arteriellen Phase folgt.

## Revendications

1. Appareil d'hémo(dia)filtration comprenant:
- un dispositif de traitement sanguin (1) comportant une première chambre (3), une seconde chambre (4) et une membrane semi-perméable (2) séparant ladite première chambre (3) de ladite seconde chambre (4), ladite première chambre ayant au moins une première ouverture (5) et une seconde ouverture (6);
- un circuit sanguin extracorporel à aiguille unique comprenant une première ligne (8) connectée à ladite première ouverture (5) et une seconde ligne (9) connectée à ladite seconde ouverture (6);
- un circuit de fluide ayant au moins une ligne de sortie (10) pour retirer le fluide usé de ladite seconde chambre (4);
- au moins une ligne de perfusion (11; 12) qui s'écoule dans ladite première ligne (8) et/ou dans ladite seconde ligne (9) pour injecter un fluide de substitution;
- une première pompe (15) agencée pour générer un premier débit (QB) de fluide dans ledit circuit sanguin;
- une seconde pompe (13; 14) agencée pour générer un second débit (QS) de fluide de substitution dans ladite ligne de perfusion (11; 12);
- des moyens d'ultrafiltration (19) agencés pour générer un troisième débit (QF) d'ultrafiltration à partir de ladite première chambre (3) vers ladite seconde chambre (4) à travers ladite membrane semi-perméable (2);
- des moyens de commande agencés pour commander ladite première pompe (15), ladite deuxième pompe (13; 14) et lesdits moyens d'ultrafiltration (19), lesdits moyens de commande comprenant des moyens pour effectuer des phases artérielles de prélèvement alternées avec des phases veineuses de retour, **caractérisées en ce que**:
* dans au moins une phase artérielle, le troisième débit QFA d'ultrafiltration est inférieur au premier débit QBA de fluide et supérieur au second débit QSA de fluide de substitution, ayant ainsi un débit QFA-QSA de prélèvement du patient;
* dans au moins une phase veineuse, le troisième débit QFV d'ultrafiltration est supérieur à zéro et inférieur à la fois au débit QBV du premier débit et au second débit QSV du fluide de substitution, ayant ainsi un débit QSV-QFV de retour au patient.

2. Appareil selon la revendication 1, dans lequel le second débit QSA de fluide de substitution dans ladite phase artérielle est inférieur au second débit QSV de fluide de substitution dans ladite phase veineuse.

3. Appareil selon la revendication 2, dans lequel le second débit QSA de fluide de substitution dans ladite phase artérielle est non nul.

4. Appareil selon l'une quelconque des revendications précédentes, dans lequel le troisième débit QFA d'ultrafiltration dans ladite phase artérielle est égal, ou presque égal, au troisième débit QFV d'ultrafiltration dans ladite phase veineuse, étant dans la l'intervalle: QFA = QFV ± 10%, ou QFA = QFV ± 20%, ou QFA = QFV ± 30%; et/ou
dans lequel le premier débit QBA de fluide dans ladite phase artérielle est égal, ou presque égal, au premier débit QBV de fluide dans ladite phase veineuse, étant dans l'intervalle: QBA = QBV ± 10%, ou QBA = QBV ± 20%, ou QBA = QBV ± 30%.

5. Appareil selon l'une quelconque des revendications précédentes, dans lequel ladite première pompe (15) est la seule pompe sanguine fonctionnant dans ledit circuit sanguin, agencée ou dans ladite première ligne (8) ou dans ladite seconde ligne (9).

6. Appareil selon l'une quelconque des revendications précédentes, dans lequel: ladite première pompe (15) est la seule pompe sanguine fonctionnant dans ledit circuit sanguin dans ladite première conduite (8) pour pomper le fluide vers ladite première chambre (3); ladite ligne d'infusion comprend une ligne de post-dilution (12) qui s'écoule dans ladite seconde ligne (9); ladite seconde pompe (14) est agencée pour fournir un écoulement de fluide de substitution dans ladite ligne de post-dilution (12).

7. Appareil selon l'une quelconque des revendications précédentes, dans lequel ladite ligne d'infusion comprend une ligne de post-dilution (12) qui s'écoule dans ladite seconde ligne (9) et une ligne de pré-dilution (11) qui s'écoule dans ladite première ligne (8); lesdits moyens de commande comprenant des moyens pour atteindre un débit de post-dilution (Qpost) dans ladite ligne de post-dilution (12) qui est supérieur dans ladite phase veineuse que dans ladite phase artérielle et/ou des moyens pour atteindre un débit de pré-dilution (Qpre) dans ladite ligne de pré-dilution (11) qui est inférieur dans ladite phase veineuse que dans ladite phase artérielle.

8. Appareil selon l'une quelconque des revendications précédentes, dans lequel ladite première pompe (15) est réversible pour la circulation du fluide dans les deux sens.

9. Appareil selon l'une quelconque des revendications précédentes, dans lequel, dans ladite phase artérielle, le premier débit QBA de fluide est supérieur à deux fois le troisième débit QFA d'ultrafiltration, et/ou dans lequel, dans ladite phase veineuse, le premier débit QBV de fluide est supérieure à deux fois le troisième débit QFV d'ultrafiltration.

10. Appareil selon l'une quelconque des revendications précédentes, dans lequel lesdits moyens de commande comprennent un mode de commande sélectionnable par l'intermédiaire d'une interface utilisateur dudit appareil, ledit mode de commande étant basé sur des durées déterminées des phases artérielles et/ou des phases veineuses, lesdites durées déterminées ayant des durées variables qui augmentent au cours d'une même séance de traitement d'hémo(dia)filtration de manière à avoir au moins une première phase artérielle avec une première durée déterminée et au moins une seconde phase artérielle avec une seconde durée déterminée supérieure à ladite première durée déterminée, ladite seconde phase artérielle étant postérieure à ladite première phase artérielle.
